# EUROPEAN PATENT APPLICATION

(11) **EP 4 512 356 A1**
(43) Date of publication of application: **26.02.2025**
(21) Application number: 24153399.1
(22) Date of filing: 23.01.2024
(51) Int. Cl.: A61B 34/30, A61B 90/70, A61L 2/00

(54) **MEDICAL INTERVENTION ASSISTANCE ROBOT**

(71) Applicant: Siemens Healthineers AG, 91301 Forchheim (DE)
(72) Inventor: Dr. Noack, Olivia, 95444 Bayreuth (DE); Lindner, Claudia, 90475 Nürnberg (DE)
(74) Representative: Siemens Healthineers Patent Attorneys

(57) **Abstract**

A medical intervention assistance robot (10) is described. The medical intervention assistance robot (10) comprises a first operating arm (1) for handling a non-sterile object, a second operating arm (2) for handling a sterile object and a support unit (3) for supporting the first operating arm (1) and the second operating arm (2). The medical intervention assistance robot (10) also comprises a control unit (7) for controlling the movement of the first operating arm (1) and the second operating arm (2). Further, a medical intervention method using a medical intervention assistance robot (10) is described.

## Description

The invention relates to a medical intervention assistance robot. The invention also refers to a medical intervention method using a medical intervention assistance robot.

In clinical routine, every single procedure needs to be prepared. For interventions, guided by medical imaging, these preparations include for example the step of cleaning and disinfection of the patient table, the step of setting up and cleaning a sterile table, equipping a table with needles, devices, skin disinfection, swabs, scalpels etc. Particularly for CT-guided interventions, the preparations also comprise the steps of setting up intervention hardware accessories, for example a joystick or a tablet holder, orienting dedicated intervention hardware and monitors and setting up sterile covers on gantry, tablet or remote control.

Typically, these preparational tasks are performed by technicians or the doctor themselves and take several minutes. Preparation basically depends on the preferences of the doctor and type of procedure to come next. Overall, the preparation can be summarized as highly repetitive and predictable tasks without direct impact on clinical outcome.

During an ideal procedure, the radiologist is supported by at least one technician. Some of those tasks performed by the technician, are being a third hand and providing flasks, meds, open packages in a way that radiologist can remain sterile.

There are even more tasks, typically performed by a technician. The tasks vary strongly in their complexity level. Typical and simple technician tasks also involve opening and closing doors, get whatever material might be used from for example drawers in the treatment room.

So far, preparation is one of the typical technician tasks, respectively also performed by interventional radiologists themselves. However, this approach is not future-proof due to increasing staff shortage in the healthcare sector. It is already observed nowadays that scanners are only used at reduced hours due to personnel lack implying less performed procedures.

It is therefore a task to provide a system for automatically assisting medical interventions of all kinds, from diagnostics to treatment or surgery.

This task is accomplished by a medical intervention assistance robot according to claim 1 and by a medical intervention method using a medical intervention assistance robot according to claim 13.

The medical intervention assistance robot according to the invention comprises a first operating arm for handling a non-sterile object, a second operating arm for handling a sterile object and a support unit for supporting the first operating arm and the second operating arm. The expression "sterile" means that the sterile object is germ-free or almost germ-free. Sterilization is normally performed using high temperature media like hot steam of hot air or using UV radiation. Compared to Sterilization, disinfection enables only a reduction of germs or pathogens. Disinfection is preferably performed using chemical disinfection substances. However, it has to be emphasized that the expressions "disinfection" and "sterilization" are widely used as a synonym in the application.

The first operating arm is preferably used for handling non-sterile objects for preparing an intervention or for supporting actions during an intervention or for completing an intervention. Preferably, the first operating arm includes a gripping unit for gripping a non-sterile object. Preferably, the gripping unit is designed with finger-like gripping tools for holding an object and for processing the object in parallel. For example, a package as a special type of an object is held with a first finger-like gripping tool and the package is opened in parallel with another finger-like gripping tool of the same operating arm. Hence, it is preferred that the gripping unit comprises at least two independent finger-like gripping tools or constructs, more preferably even three such finger-like gripping tools or constructs. The gripping unit is preferably fixed to the first operating arm. Alternatively, the gripping unit is arranged to be easily removable from the first operating arm. It is preferred that the first operating arm is designed such that it is cleanable very well and such that it is constructed based on hygienic design principles.

The second operating arm is preferably used for handling sterile objects for preparing an intervention or for supporting actions during an intervention or for completing an intervention. For handling sterile objects, the second operating arm preferably comprises a sterile gripping unit. Preferably, the sterile gripping unit is designed to be easily removable from the second operating arm. The second operating arm preferably comprises a surface material which is easily cleanable and disinfectable or even sterilizable.

Preferably, the first operating arm and the second operating arm comprise actor units for moving them to a predetermined position or pose.

The medical intervention assistance robot according to the invention further comprises a control unit for controlling the movement of the first operating arm and the second operating arm. The control unit is preferably designed to transmit control orders to the actor units of the first operating arm and the second operating arm for moving the first operating arm and the second operating arm to a predetermined position or pose.

Preferably, the control unit is designed to control an interplay or an interaction between the first operating arm and the second operating arm. In particular, such an interaction can be advantageously performed by using the sterile second operating arm for holding a sterile package and by using the first non-sterile operating arm for putting a non-sterile object into a package or for removing the non-sterile object from the package. Such, an object, in particular an accessory, can be automatically packed or removed without any need of human support.

Preparation may comprise one of the following types of preparation action:
- a preparation of an imaging modality, in case the medical imaging modality is used for guiding an intervention, in particular such a medical imaging modality may comprise a CT scan unit or an MRT scan unit or an angiography scan unit, in particular an angiography CT scan unit, or an ultrasonic scan unit, respectively,
- a procedure preparation for preparing the intervention procedure,
- in particular cleaning and disinfection the patient table,
- positioning the patient at the correct position on the patient table,
- finalizing the intervention set-up,
- supporting the patient with sterile draping,
- supporting the interventionalist with dressing sterile.

Typical supporting actions during an intervention comprise:
- providing an autonomous sterile table,
- assisting the interventionalist during the intervention procedure,
- non-sterile interaction with environment.

Typical actions for completing an intervention comprise:
- clearing the scan unit of an imaging modality, in case such a scan unit, in particular one of the above-mentioned special types, is used for guiding intervention,
- cleaning the scan unit, in case a scan unit is used for guiding intervention,
- unmounting equipment and bring back the equipment to stock,
- emptying a sterile table,
- cleaning a patient table,
- cleaning a gantry,
- cleaning a sterile table.

For example, for preparation, the first operating arm may be used for some henchman tasks like cleaning a table, in particular a patient table, or a gantry using a spray and/or a wipe. The first operating arm may also be used for fetching and/or setting up required need for interventions regardless of modality or in case of the use of a CT-system, accessories. Such need for interventions may comprise incidentals like swabs, needles, medications, disinfectants, in particular povidone-iodine, plasters or scalpels. The first operating arm may also be used for aligning hardware components or for passing or holding local anesthesia flasks or other types of med flasks or for opening packages.

Further, the first operating arm may be used during the intervention procedure for holding syringes, flasks, for unpacking disposables and for mixing medication by shaking.

Further, the first operating arm can be used for opening or closing a door or for changing light settings or for operating non-covered components, for example an ablation generator. Furthermore, the first operating arm is preferably used for removing covers or for unmounting accessories or for cleaning them.

Preferably, the first operating arm is used for getting positioning aids for positioning a patient to be examined. These are differently shaped cushion-like cushions so that the patient can be positioned in a stable position. These are usually stored on shelves in the room or rarely in the neighboring room (if not needed).

The second operating arm is preferably used as a third hand for sterile operations. In particular, the third operating arm is used for equipping a sterile table, for assistance during procedure or for sterile covering the patient or an object. The second operating arm is also used for unpacking a coat or gloves or for being a third hand to hold the one-way cloths in proper position for the doctor.

Further, the second operating arm is preferably used for setting-up or for disinfecting a sterile table and/or for equipping the sterile table with needles or devices or skin disinfectant or swabs or scalpels.

Preferably, the control unit is also used for synchronized controlling the movement of the first operating arm and the second operating arm such that the first operating arm and the second operating arm work together with each other. For example, the first operating arm holds a disinfection flask for disinfection of a surface or for disinfecting a human injection site in particular using iodine. The second operating arm performs all actions comprising the contact with objects or surfaces which may be touched by the sterile gloves of the doctor or interventionalist after a contact with the second operating arm. Interplay of both robotic arms is preferably needed for sterile packing of components, for unpacking and providing flasks and for supporting interventional radiologist in dressing sterile.

Advantageously, the availability of an intervention assistance robot is higher than the availability of a human technician. Further, the intervention assistance robot reduces the regular costs for salary of employees. Generally, the number of the needed persons for an intervention is reduced due to the automatic assistance. The combination of a first operating arm for handling a non-sterile object and a second arm for handling a sterile object enables to automatically carry out transport actions and unpacking and packing actions concerning sterile objects packed in non-sterile covers. Advantageously, also an interplay of both robotic arms is enabled, in particular for sterile packing of components like a tablet or a remote control and/or for unpacking and/or providing objects like flasks.

According to the method for using a medical intervention assistance robot, which is preferably a medical intervention assistance robot according to the invention, according to the invention, a first operating arm for handling a non-sterile object is used and a second operating arm for handling a sterile object is used, wherein the first operating arm and the second operating arm are supported using a support unit. Advantageously, non-sterile objects can be handled without contaminating the second arm dedicated to handling sterile objects.

Some units or modules of the medical intervention assistance robot mentioned above can be completely or partially realized as software modules running on a processor of a respective computing system, e.g. of the control unit of the medical intervention assistance robot. A realization largely in the form of software modules can have the advantage that applications already installed on an existing computing system can be updated, with relatively little effort, to install and run these units of the present application. The object of the invention is also achieved by a computer program product with a computer program that is directly loadable into the memory of a computing system, in particular a control unit, and which comprises program units to perform the step of controlling the movement of the first operating arm and the second operating arm of the inventive method for using the first operating arm and the second operating arm, when the program is executed by the computing system. In addition to the computer program, such a computer program product can also comprise further parts such as documentation and/or additional components, also hardware components such as a hardware key (don-gle etc.) to facilitate access to the software.

A computer readable medium such as a memory stick, a hard-disk or other transportable or permanently-installed carrier can serve to transport and/or to store the executable parts of the computer program product so that these can be read from a processor unit of a computing system. A processor unit can comprise one or more microprocessors or their equivalents.

The dependent claims and the following description each contain particularly advantageous embodiments and developments of the invention. In particular, the claims of one claim category can also be developed analogously to the dependent claims of another claim category. In addition, within the scope of the invention, the various features of different exemplary embodiments and claims can also be combined to form new exemplary embodiments.

Preferably, the intervention for which the medical intervention assistance robot is used, comprises an image-guided intervention. For imaging, a medical imaging system may be used, in particular one of the following types: a computer tomography system, a magnetic resonance imaging system, an ultrasound system, an X-ray system, in particular a planar X-ray system or an angiography system or a C-arm system or an angiography CT system. The intervention assistance robot can alternatively be used for blind interventions, for which sterility needs to be maintained and sterile equipment must be prepared. Advantageously, the interventionalist is enabled to perform interventions autonomously in an efficient way.

In a variant of the medical intervention assistance robot according to the invention, the medical intervention assistance robot according to the invention comprises a mobility unit for mobilizing the robot. Advantageously, the mobility unit enables the robot to be mobile such that the robot can transport required objects over longer distances and is able to save the doctor from having to walk around the treatment room or outside the treatment room himself. The mobility unit enables re-stocking, fetching and/or storing accessories etc.

Preferably, the mobility unit includes rolling elements for moving in all directions. Rolling elements, particularly wheels, enable to move a robot with low energy expense and very smooth without any shock such that equipment being transported on the robot does not fall down from the robot.

Particularly preferred, the rolling elements comprise one of the following types:
- a Mecanum wheel,
- an all-side wheel.

Advantageously, the mentioned types of wheels enable to change the moving direction abruptly and with limited space for the change of the moving direction.

The intervention assistance robot according to the invention preferably includes a sterilization chamber. Advantageously, a sterile condition of the second operating arm can be assured in every situation.

Further, the medical intervention assistance robot according to the invention includes a sterile table, preferably on the top of the support unit of the medical intervention assistance robot. Preferably, the sterile table is designed to be attached to a surgical rail, in particular at a scanner, so that the medical intervention assistance robot can move freely during interventions.

Most patient tables have mounted rails on the sides, these are typically made of stainless steel and are used to attach things to them. There are not any number of different variants of these rails, but rather a standard has emerged, which is referred to as "surgical rail".

Preferably, the sterile table comprises as material stainless steel. Advantageously, the table is inert against disinfection fluids.

Preferably, the sterile table or the sterile chamber is arranged to keep an object sterile based on one of the following methods:
- using a disinfection spray,
- using a wipe,
- radiating with UV light.

Radiating with UV light enables to disinfect or even sterilize an object without contacting with a sterilization or disinfection agent or means. For example, the sterilization or disinfection agent may cause an allergic reaction. Using a wipe enables to mechanically clean an object additionally to a sterilization or disinfection of the object.

For example, the sterilization or disinfection can be performed using the first and second operating arms, wherein the first operating arm holds the disinfection or sterilization spray and the object to be disinfected or sterilized is positioned on the sterile table. In an alternative variant, the object to be disinfected or even to be sterilized is positioned in the sterile chamber and is disinfected or even sterilized therein, preferably using a UV light, in particular UV-C light.

The medical intervention assistance robot according to the invention further preferably includes a storage section. Advantageously, objects necessary or useful for an intervention can be stored in the storage section.

Preferably, the first operating arm of the medical intervention assistance robot according to the invention is arranged to execute any of the following handling functions:
- fetching an object,
- mounting an object,
- orienting an object,
- holding an object.

Advantageously, a doctor tasked with carrying out an intervention, is exonerated from procuring and preparing an object being necessary for the intervention.

Also preferably, the first operating arm of the medical intervention assistance robot according to the invention is arranged to execute one of the following actions:
- cleaning a patient table,
- cleaning a gantry,
- getting a disposables from the stock,
- open or close a door,
- passing or holding a local anesthetic or a med flask,
- open a package,
- interacting with a non-sterile covered part of an imaging system,
- interacting with a non-sterile covered machine of a type being different from an imaging system,
- supporting the interventionalist to become sterile.

Advantageously, a doctor can concentrate completely on the actual intervention, since the intervention assistance robot takes over all the henchman's work for him.

The second operating arm of the medical intervention assistance robot according to the invention is preferably arranged to execute one of the following actions:
- covering a gantry,
- packing a tablet and/or a remote control in a sterile bag,
- supporting with covering a patient.

Packing tablets and/or remote controls in sterile bags is performed by an interaction of the first operating arm and the second operating arm, since the bags are sterile only on the outside and non-sterile on the inner side just as the object inside the bags.

Advantageously the environment of the intervention is kept sterile so that an infection of a patient can be avoided.

The control unit of the medical intervention assistance robot according to the invention is preferably arranged to function based on one of the following communication principles:
- gesture recognition,
- voice recognition,
- a touch display interaction function.

Advantageously, the intervention assistance robot is enabled to be controlled by a doctor tasked with an intervention using common means of communication for control.

Further, the intervention assistance robot optionally comprises technical means for exploring the environment and for enabling communication with a human person. In particular, these technical means include sensor means or communication means. Preferably these technical means comprise at least one of the following types of technical means:
- a sensor unit,
- a camera,
- a display,
- speakers.

Advantageously, the sensor unit and the camera can be used for perceiving the surroundings of the intervention assistance robot, in particular for localizing and gripping an object or for driving through the treatment room without any collision.

The display and the speakers can be used for transmitting information by the intervention assistance robot to the intervening doctor or vice versa. For example, the intervention assistance robot is enabled to communicate with the intervening doctor such that the assistance robot provides the doctor with a required object.

The invention is explained below with reference to the figures enclosed once again. The same components are provided with identical reference numbers in the various figures.

The figures are usually not to scale.
- FIG 1: shows a schematic illustration of a medical intervention assistance robot according to an embodiment of the invention,
- FIG 2: shows a flow chart, illustrating a medical intervention method using an assistance robot according to an embodiment of the invention.

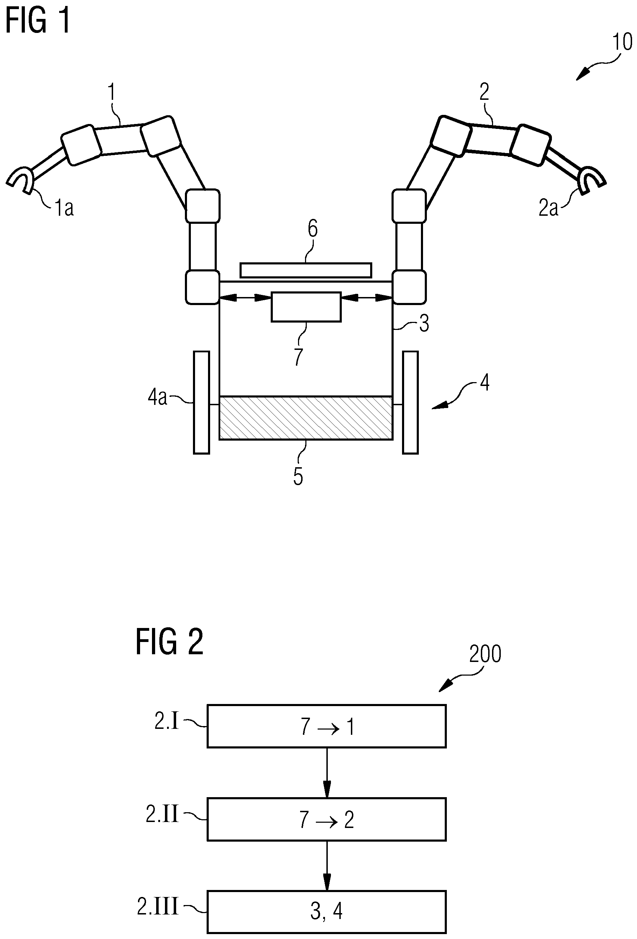

In FIG 1, a schematic illustration of a medical intervention assistance robot 10 according to an embodiment of the invention is depicted. The medical intervention assistance robot 10 comprises a first operating arm 1 for handling a non-sterile object. The first operating arm 1 is illustrated on the left side of FIG 1. The first operating arm 1 comprises a gripping unit 1a for gripping non-sterile objects. Further, the medical intervention assistance robot 10 comprises a second operating arm 2 for handling a sterile object. The second operating arm 2 is illustrated on the right side of FIG 1. The second operating arm 2 comprises a gripping unit 2a for gripping a sterile object. The medical intervention assistance robot 10 also comprises a support unit 3 for supporting the first operating arm 1 and the second operating arm 2. The support unit 3 is illustrated in the center of FIG 1. Part of the support unit 3 is also a mobility unit 4 including wheels 4a for changing the position of the medical intervention assistance robot 10. On the top of the support unit 3, a sterile table 6 is arranged for transporting sterile objects which can mainly be handled by the second operating arm 2. The support unit 3 also comprises a sterilization chamber 5 for implementing a sterilization process for the sterile table 6 or the sterile part of the robotic arm, i.e. the gripping unit 2a of the second operating arm 2. The sterilization process is realized using disinfection chemicals like ethanol and UV light. Further, the medical intervention assistance robot 10 comprises a control unit 7 for controlling the movement of the first operating arm 1 and the second operating arm 2.

In FIG 2 a flow chart 200, illustrating a medical intervention method using a medical intervention assistance robot 10 according to an embodiment of the invention is shown.

In step 2.I, a control order is emitted by a control unit 7 of a medical intervention assistance robot 10 for controlling a first operating arm 1 or gripping a package whose outer surface is non-sterile. The package contains a sterile object, for example a scalpel. The first operating arm 1 is also used for opening the package.

In step 2.II, a control order is emitted by the control unit 7 of the medical intervention assistance robot 10 for controlling a second operating arm 2 for handling a sterile object for removing the scalpel from the package. It has to be mentioned that the step 2.1 and step 2.II can be carried out at the same time or sequentially.

In step 2.III, the supporting unit 3 of the medical invention assistance robot 10, including a mobility unit 4, is used for transporting objects for a pre- and post-preparation step and for transporting objects for the intervention itself, like a scalpel, which is kept hold of the second operating arm 2, to a doctor standing at an operating table.

In an alternative scenario, the doctor or interventionalist takes everything he can easily handle with one hand from the sterile table 6 of the robot 1. In the alternative scenario, the doctor or interventionalist only changes his position very rarely during the most types of intervention, i.e. driving behind the doctor or interventionalist is not frequently needed in most scenarios.

In these scenarios, the mobile support of the robot relates more to the pre- and post-preparation step or when supplies are needed or interaction with the environment is required.

Further, there is another use case in which the robot could offer added value if it delivers in a sterile manner, possibly with a change of location, namely when doctor progresses the needle partially outside and inside of the bore for a CT-guided needle intervention.

This means that the patient table for the needle advance is not moved out of the gantry, but rather the doctor or interventionalist leans into it. The reasons are either to save time or because live imaging (fluoroscopy) is required.

Finally, it should be pointed out once again that the detailed methods and structures described above are exemplary embodiments and that the basic principle can also be varied in wide areas by the person skilled in the art without leaving the scope of the invention, insofar as it is specified by the claims. For the sake of completeness, it should also be noted that the use of the indefinite articles "a" or "an" does not exclude the fact that the characteristics in question can be present multiple times. Likewise, the term "unit" does not exclude the fact that it consists of several components, which may also be spatially distributed. Further, independent of the grammatical term usage, individuals with male, female or other gender identities are included within the term.

## Claims

1. Medical intervention assistance robot (10), comprising:
- a first operating arm (1) for handling a non-sterile object,
- a second operating arm (2) for handling a sterile object,
- a support unit (3) for supporting the first operating arm (1) and the second operating arm (2),
- a control unit (7) for controlling the movement of the first operating arm (1) and the second operating arm (2).

2. Medical intervention assistance robot according to claim 1, comprising a mobility unit (4) for mobilizing the robot (10) .

3. Medical intervention assistance robot according to claim 2, wherein the mobility unit (4) includes rolling elements (4a) for moving in different directions.

4. Medical intervention assistance robot according to claim 3, wherein the rolling elements (4a) comprise one of the following types:
- a Mecanum wheel,
- an all-side wheel.

5. Medical intervention assistance robot according to anyone of the preceding claims, further including a sterilization chamber (5).

6. Medical intervention assistance robot according to anyone of the preceding claims, further including a stainless steel sterile table (6) on the top of the support unit (3).

7. Medical intervention assistance robot according to claim 6, wherein the sterile table (6) is arranged to keep an object sterile based on at least one of the following methods:
- using a disinfection spray,
- using a wipe,
- radiating with UV light.

8. Medical intervention assistance robot according to anyone of the preceding claims, further including a storage section.

9. Medical intervention assistance robot according to anyone of the preceding claims, wherein the first operating arm (1) is arranged to execute at least one, preferably any, of the following handling functions:
- fetching an object,
- mounting an object,
- orienting an object,
- holding an object.

10. Medical intervention assistance robot according to anyone of the preceding claims, wherein the first operating arm (1) is arranged to execute at least one of the following actions:
- cleaning a patient table,
- cleaning a gantry,
- getting disposables from the stock,
- open or close a door,
- passing or holding a local anesthetic or a med flask,
- open a package,
- interacting with a non-sterile covered part of an imaging system,
- interacting with a non-sterile covered machine of a type being different from an imaging system,
- supporting an interventionalist to become sterile.

11. Medical intervention assistance robot according to anyone of the preceding claims, wherein the second operating arm (2) is arranged to execute at least one of the following actions:
- covering a gantry,
- packing a tablet and/or a remote control in a sterile bag,
- supporting with covering a patient.

12. Medical intervention assistance robot according to anyone of the preceding claims, wherein the control unit is controlled based on one of the following communication principles:
- gesture recognition,
- voice recognition,
- a touch display interaction function.

13. Medical intervention method using a medical intervention assistance robot, preferably a medical intervention assistance robot (10) according to anyone of the preceding claims, comprising the steps:
- using a first operating arm (1) for handling a non-sterile object,
- using a second operating arm (2) for handling a sterile object,
- supporting the first operating arm (1) and the second operating arm (2) using a support unit (3),
- controlling the movement of the first operating arm (1) and the second operating arm (2) using a control unit.

14. A computer program product comprising instructions which, when the program is executed by a computer, cause the computer to carry out the step of controlling the movement of the first operating arm (1) and the second operating arm (2) of the method according to claim 13.

15. A computer-readable storage medium comprising instructions which, when executed by a computer, cause the computer to carry out the step of controlling the movement of the first operating arm (1) and the second operating arm (2) of the method according to claim 13.
